# EUROPÄISCHE PATENTSCHRIFT

(11) **EP 0 526 669 B1**
(45) Veröffentlichungstag und Bekanntmachung des Hinweises auf die Patenterteilung: **06.03.1996**
(21) Anmeldenummer: 91120747.0
(22) Anmeldetag: 03.12.1991
(51) Int. Cl.: A61M 25/00, A61D 19/02, A61B 17/435

(54) **Instrumentensatz für den uterinen Embryonentransfer und für die intrauterine Insemination**
Instrument set for the uterus embryo transfer and intrauterine insemination
Jeu d'instruments pour le transfert d'embryons utérins et pour l'insémination intrautérine

(30) Priorität: 25.06.1991 DE 9107792 U
(43) Veröffentlichungstag der Anmeldung: 10.02.1993
(73) Patentinhaber: LABOTECT-LABOR-TECHNIK, GÖTTINGEN, GMBH, D-37120 Bovenden (DE)
(72) Erfinder: Schinkel, Otto, W-3406 Bovenden (DE); Horvath, Josef, W-3400 Göttingen (DE)
(74) Vertreter: Rehberg, Elmar, Dipl.-Ing.

(56) Entgegenhaltungen:
- EP-A- 0 046 485
- DE-A- 3 820 213
- DE-C- 94 003
- DE-C- 443 112
- FR-A- 2 635 453

## Beschreibung

Die Erfindung bezieht sich auf einen Instrumentensatz für den uterinen Embryonentransfer und die intrauterine Insemination mit einem Katheterführungsrohr und einem in seinem proximalen Bereich mit einem Verstärkungsrohr aus Metall versehenen Katheter. Ein derartiger Instrumentensatz wird verwendet, wenn in vitro befruchtete Eizellen nach in der Regel zweimaliger Zellteilung in den Uterus überführt, bzw. wenn Samenzellen in den Uterus übertragen werden.

Ein Instrumentensatz der eingangs beschriebenen Art ist bekannt. Er weist ein gerades, an seinem distalen Ende abgerundetes Katheterführungsrohr auf. Bei dem zugehörigen Katheter ist das Verstärkungsrohr innen angeordnet, so daß es im proximalen Bereich die Innenoberfläche des geraden Katheters ausbildet. Beim Einführen in den Uterus wird dieses bekannte einstückig aus Kunststoff und flexibel ausgebildete Katheterführungsrohr durch einen Mandrin aus Metall stabilisiert. Der Mandrin erlaubt, dem Katheterführungsrohr vor dem Einführen in die Gebärmutter eine das Einführen erleichternde Form zu geben. Diese Form behält das Katheterführungsrohr natürlich nur solange bei, wie der Mandrin in seinem Inneren angeordnet ist. Das Verstärkungsrohr verhindert ein Umbiegen des Katheters durch die Einwirkung der Gewichtskraft einer in den Spritzenanschluß des Katheters eingesetzten Spritze. Als nachteilig stellt sich bei diesem bekannten Instrumentensatz heraus, daß beim Einführen des Katheterführungsrohrs in die Gebärmutter Verletzungen des Endometriums kaum zu verhindern sind. Das distale Ende des Katheterführungsrohrs zerfurcht das Endometrium und löst Blutungen aus. Beim eigentlichen Embryonentransfer stellt sich heraus, daß von der Oberfläche des Verstärkungsrohrs eine nicht unbeachtliche Verletzungsgefahr für die Embryonen ausgeht. Dies ist auf die relativ rauhe Oberfläche des aus Metall gezogenen Verstärkungsrohrs zurückzuführen. Da auch bei einem verletzten Endometrium kein erfolgreicher Embryonentransfer durchzuführen ist, muß die Erfolgsquote bei der Verwendung dieses bekannten Instrumentensatzes als äußerst gering bezeichnet werden.

Bei einem Instrumentensatz für den uterinen Embryonentransfer, der sich von der eingangs beschriebenen Art dadurch unterscheidet, daß der Katheter kein Verstärkungsrohr aus Metall aufweist, sondern in seinem proximalen Bereich nur durch zwei coaxial angeordnete Schläuche stabilisiert ist, ist ein Katheterführungsrohr mit einer eichelförmigen Spitze vorgesehen. Dieses Katheterführungsrohr ist bei Verwendung eines flexiblen Mandrins auch ohne Vorformung in die Gebärmutter einführbar.

Hierdurch ist auch das Katheterführungsrohr beim Einführen verhältnismäßig flexibel und die Gefahr von Verletzungen des Endometriums ist reduziert. Andererseits besteht die nicht geringe Wahrscheinlichkeit, daß gerade das eichelförmige distale Ende des Katheterführungsrohr verletzend in das Endometrium eindringt. Hierbei ist besonders die scharfe Kante zwischen der auslaufenden Oberfläche der Eichel und der Austrittsöffnung des Katheters von Bedeutung. Nachteilig wirkt sich natürlich auch das Fehlen eines echten Verstärkungsrohrs für den Katheter aus. Trotz der zwei coaxial angeordneten Schläuche kann der Katheter durch Einwirken der Gewichtskraft einer angesetzten Spritze umbiegen und dabei zerstört oder unkontrolliert aus dem Katheterführungsrohr herausgezogen werden. Beide Möglichkeiten sind bei der Durchführung eines uterinen Embryonentransfers, bzw. einer intrauterinen Insemination selbstverständlich äußerst unerwünscht.

Bei dem bekannten Instrumentensatz weist das Katheterführungsrohr in seinem distalen Bereich einen verschiebbaren, zur Anlage an die Portio vorgesehenen Anschlag auf.

Ein Instrumentensatz für den transvaginalen Gametentransfer und für die Tubenkatheterisierung weist ein Katheterführungsrohr mit einer Krümmung im distalen Bereich auf, dessen distales Ende mit einer Kugel abschließt. Die Krümmung ist zur Erleichterung des Eintritts des Katheterführungsrohrs in den Tubeneingang vorgesehen. Die Kugel bewirkt die Zentrierung einer Austrittsöffnung für den Katheter im Tubus.

Als Material für Embryonentransferkatheter und die zugeordneten Katheterführungsrohre ist Teflon bekannt. Teflon kann zur Ausbildung vorteilhaft glatter Oberflächen herangezogen werden.

Der Erfindung liegt die Aufgabe zugrunde, einen Instrumentensatz der eingangs beschriebenen Art aufzuzeigen, bei dessen Verwendung der Embryonentransfer bzw. die Insermination mit einer möglichst hohen Erfolgsquote verbunden ist.

Erfindungsgemäß wird dies dadurch erreicht, daß das Katheterführungsrohr in seinem distalen Bereich eine leichte Krümmung aufweist, daß das Katheterführungsrohr an seinem distalen Ende mit einer Kugel abschließt und daß das Verstärkungsrohr aus Metall den Katheter umschließt. Überraschenderweise stellt sich heraus, daß auch bei einem Katheterführungsrohr eines Instrumentensatzes für den uterinen Embryonentransfer bzw. für die intrauterine Insemination eine leichte Krümmung im distalen Bereich vorteilhaft ist. Sie erleichtert das Einführen des Katheterführungsrohrs in den Uterus. So kann aufgrund der Krümmung bei dem neuen Instrumentensatz gänzlich auf einen Mandrin verzichtet werden. Die Flexibilität des Katheterführungsrohrs ist damit vollständig zum Erreichen der gewünschten Endlage und zur Vermeidung von Verletzungen des Endometriums insbes. bei Vorliegen einer Hyperflexio uteri oder eines zerklüfteten Zervikalkanals nutzbar. Hierbei wirkt sich aber auch die kugelförmige Ausbildung des distalen Endes des Katheterführungsrohrs positiv aus. Die Kugel gleitet mit minimaler Verletzungsgefahr über das Endometrium hinweg. Zudem wird die Ausbildung einer schabenden Kante zwischen der Außenoberfläche und der Innenoberfläche des Katheterführungsrohrs durch die Kugel weitgehend verhindert. Die distale Öffnung des Katheterführungsrohrs für den Katheter kommt mit dem Endometrium in der Regel gar nicht in Berührung. Hierbei spielt natürlich auch die gekrümmte Ausbildung des distalen Bereichs eine Rolle. Wesentlich für die Erhöhung der Erfolgsquote bei der Verwendung des neuen Instrumentensatzes ist aber auch die Anordnung des Verstärkungsrohrs um den Katheter herum. Die Embryonen kommen so mit dem gezogenen Metall des Verstärkungsrohrs nicht in Berührung. Sie gleiten ohne Verletzungsgefahr an der glatten Innenwand des Katheters entlang.

Das Katheterführungsrohr kann einstückig aus Teflon und insbesondere in seinem distalen Endbereich flexibel ausgebildet sein. Auf diese Weise läßt sich ohne Aufwand ein Katheterführungsrohr für den einmaligen Gebrauch ausbilden, das dennoch allen notwendigen Anforderungen entspricht.

Der Außendurchmesser der Kugel kann mehr als 2-mal, vorteilhafterweise 3 bis 4-mal so groß sein wie der Innendurchmesser des Katheterführungsrohrs an dessen distaler Öffnung. Die Kugel, mit der das Katheterführungsrohr abschließt, muß eine Mindestgröße aufweisen, um ohne dieses zu verletzen über das Endometrium gleiten zu können. Andererseits erschwert ein zu großer Durchmesser das Einführen des Katheterführungsrohrs in den Uterus. Bei den üblichen Außendurchmessern der verwendeten Katheter und dem entsprechenden Innendurchmesser des Katheterführungsrohrs erweist sich eine Kugel mit einem Durchmesser, der gut 3-mal so groß ist wie der Innendurchmesser des Katheterführungsrohrs als idealer Abschluß des Katheterführungsrohrs.

Der Katheter kann in seinem distalen Bereich eine der Krümmung des Katheterführungsrohrs entsprechende Krümmung aufweisen. Die zusätzliche Krümmung des Katheters erleichtert dessen verletzungsfreies Vorschieben über das distale Ende des gekrümmten Katheterführungsrohrs hinaus.

Der Katheter kann in seinem proximalen Bereich eine Skala aufweisen, die in Verbindung mit der proximalen Öffnung des Katheterführungsrohrs die relative Lage des distalen Endes des Katheters zu dem distalen Ende des Katheterführungsrohrs anzeigt. Auf diese Weise läßt sich auch die Verletzung des Endometriums durch einen zu weit über die Kugel des Katheterführungsrohrs hinausgeschobenen Katheter vermeiden.

Das Verstärkungsrohr kann aus Edelstahl ausgebildet und ortsfest in dem Spritzenanschluß des Katheters gelagert sein. Der Spritzenanschluß muß sowieso mit dem Schlauch des Katheters fest verbunden werden. Hierbei kann gleichzeitig die unkomplizierte Lagerung des Verstärkungsrohrs erfolgen. Die Verwendung von Edelstahl für das Verstärkungsrohr ist unkompliziert und schließt Korrosionsprobleme aus.

Der Katheter kann einen Schlauch aus poliertem Kunststoff aufweisen und an seinem distalen Ende abgerundet ausgebildet sein. Die Verwendung von Kunststoff für den Schlauch des Katheters stellt dessen Strahlenbeständigkeit und damit die Möglichkeit seiner Sterilisation durch Bestrahlung sicher. Gegenstände aus Teflon zerfallen demgegenüber bei Bestrahlung. Die polierten Oberflächen des Kunststoffs stellen für die Außenhaut der Embryonen keine Gefahr dar. Die abgerundete Spitze des Katheters dient wiederum dem Schutz des Endometriums.

Auf der Außenseite des Katheterführungsrohrs kann in seinem distalen Bereich eine Skala und eine auf der Skala verschiebbare Marke vorgesehen sein. Die verschiebbare Marke kann als Anschlag an der Portio verwendet werden, um bestimmte, an der Skala ablesbare Eindringtiefen des Katheterführungsrohrs in dem Uterus zu erreichen. Vorteilhafterweise ist die Marke auch geeignet die Richtung der Krümmung anzuzeigen. Dies gestattet dem den Instrumentensatz einsetzenden Arzt, das Katheterführungsrohr auch nach Einführen der Krümmung in den Uterus gezielt zu führen.

Das Katheterführungsrohr kann an seinem proximalen Ende eine Kupplungshülse aufweisen, wobei der Katheter einen Spritzenanschluß mit einem Gegenstück zum Eingriff in die Kupplungshülse aufweist. So sind das Katheterführungsrohr und der Katheter zur vereinfachten Handhabung vor dem Einführen miteinander verbindbar. Ferner ist eine Kupplungshülse aus weichem Kunststoff zugleich ein ideales Griffstück für das Katheterführungsrohr.

Das Katheterführungsrohr kann zur Aufnahme in einer Halterung eines Spekulums mit Kugelzange vorgesehen sein. Dies gestattet die definierte Lagerung des Katheterführungsrohrs gegenüber dem Uterus und hält dem den Instrumentensatz einsetzenden Arzt die Hände, beispielsweise zum Einführen des Katheters frei.

Die Erfindung wird im Folgenden anhand eines Ausführungsbeispiels näher erläutert und beschrieben. Es zeigt:
- Figur 1: das Katheterführungsrohr,
- Figur 2: den Katheter mit Verstärkungsrohr und
- Figuren 3 bis 5: den Instrumentensatz im Einsatz.

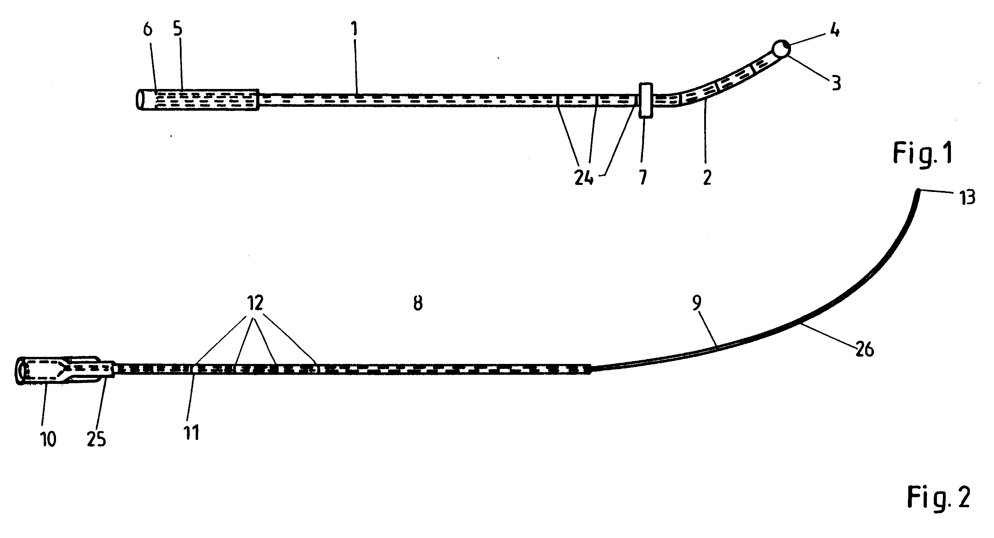

Das in Figur 1 dargestellte Katheterführungsrohr 1 weist in seinem distalen Bereich eine leichte Krümmung 2 auf und schließt an seinem distalen Ende in einer Kugel 3 ab. Der Außendurchmesser der Kugel 3 beträgt hier das 3,5-fache des Innendurchmessers des Katheterführungsrohrs 1 an seiner distalen Öffnung 4. An seinem proximalen Ende weist das Katheterführungsrohr 1 eine Kupplungshülse 5 auf, die zugleich ein Griffstück für das Katheterführungsrohr 1 bildet. Die proximale Öffnung 6 des Katheterführungsrohrs 1 und/oder die Kupplungshülse 5 sind angetrichtert ausgebildet, um das Einführen eines Katheters zu erleichtern. Das Katheterführungsrohr 1 samt Kugel 3 aber ohne das Kupplungsstück 5 ist einstückig aus flexiblem Teflon ausgebildet. Besondere Flexibilität weist das Katheterführungsrohr 1 hierbei in seinem distalen Bereich mit der Krümmung 2 auf. Das Kupplungsstück 5 besteht aus abrutschsicherem weichen Kunststoff. In seinem distalen Bereich weist das Katheterführungsrohr 1 eine Skala 24 und eine auf der Skala verschiebbare Marke 7 auf. Die Marke 7 ist als Anschlag vorgesehen und zeigt ferner die Richtung der Krümmung 2 an. Ein Mandrin ist für das Katheterführungsrohr 1 normalerweise nicht vorgesehen.

Der in Figur 2 dargestellte Katheter 8 weist einen Schlauch 9 und an seinem proximalen Ende einen Spritzenanschluß 10 auf. Der Schlauch 9 besteht aus einem polierten Kunststoff, der die Ausbildung extrem glatter Oberflächen erlaubt. Im proximalen Bereich des Katheters 8 wird der Schlauch 9 von einem Verstärkungsrohr 11 umschlossen. Außerhalb des Verstärkungsrohrs 11 im distalen Bereich des Katheters 8 ist der Schlauch 9 analog zur Krümmung 2 des Katheterführungsrohrs 1 mit einer Krümmung 26 versehen. Das Verstärkungsrohr 11 ist aus Edelstahl gezogen und gemeinsam mit dem Schlauch 9 in dem Spritzenanschluß 10 gelagert. Das Verstärkungsrohr 11 bildet hierbei an keiner Stelle die Innenoberfläche des Katheters 8. In seinem proximalen Bereich weist der Katheter 8 auf der Außenseite des Verstärkungsrohrs 11 eine Skala 12 auf. Die Skala 12 zeigt in Verbindung mit der proximalen Öffnung 6 des Katheterführungsrohrs 1 gemäß Figur 1 die relative Lage des distalen Endes 13 des Katheters 8 gegenüber der distalen Öffnung 4 des Katheterführungsrohr 1 an. Der Spritzenanschluß 10 weist ein Gegenstück 25 zum Eingriff in die Kupplungshülse 5 des Katheterführungsrohrs 1 auf. Der Katheter 8 ist ebenso wie das Katheterführungsrohr 1 gemäß Figur 1 als steril verpackter Artikel zum einmaligen Gebrauch bestimmt.

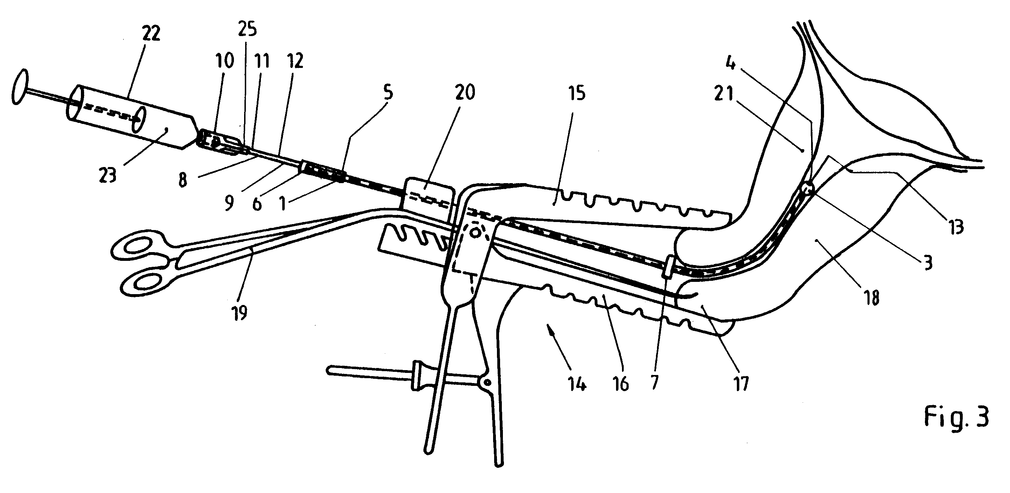

Figur 3 zeigt den neuen Instrumentensatz mit dem Katheterführungsrohr 1 und dem Katheter 8 beim uterinen Embryonentransfer. An einem in die hier nicht dargestellten Scheide eingeführten Spekulum 14 mit Oberlöffel 15 und Unterlöffel 16 ist eine die Portio 17 des Uterus 18 ergreifende und vorziehende Kugelzange 19 eingehängt. An der Kugelzange 19 ist eine Halterung 20 vorgesehen. In der Halterung 20 ist das Katheterführungsrohr 1 eingeklemmt gelagert. Das Katheterführungsrohr 1 ragt beim Anschlag der Marke 7 an die Portio 17 etwa 4 cm in den Uterus hinein, wobei dieser Wert durch Verschieben der Marke 7 einstellbar ist. Die Kugel 3 am distalen Ende des Katheterführungsrohrs 1 vermeidet beim Einführen des Katheterführungsrohrs 1 in den Uterus 18 eine Verletzung des Endometriums 21. Die Oberfläche der Kugel 3 fungiert hierbei als breitflächige Abstützung der auf das Katheterführungsrohr 1 vom Arzt ausgeübten Vorschubkraft. Hilfreich beim Einführen des Katheterführungsrohrs 1 in den Uterus 18 ist auch die Krümmung 2 gemäß Figur 1. Die ursprüngliche Krümmung 2 ist in Figur 3 nicht mehr zu erkennen, da sich das Katheterführungsrohr 1 den Formen des Uterus 18 angepaßt hat. Der Katheter 8 im Inneren des Katheterführungsrohrs 1 steht mit seinem distalen Ende 13 etwa 1 - 2 cm über die distale Öffnung 4 an der Kugel 3 des Katheterführungsrohrs 1 hervor, wobei sich der Zahlenwert wiederum auf eine typische Gesamtlänge des Uterus 18 von ca. 6,5 cm bezieht. Das Maß des Vorstehens kann an der Skala 12 gegenüber der proximalen Öffnung 6 des Katheterführungsrohrs 1 abgelesen werden. In den Spritzenanschluß 10 des Katheters 8 ist eine Spritze 22 eingesetzt. In der Spritze 22 befindet sich eine Flüssigkeit 23, die den zu übertragenden Embryo enthält. Daß der Schlauch 9 des Katheters 8 unter Einwirkung der Gewichtskraft der Spritze 22 abknickt oder aus dem Katheterführungsrohr 1 herausgezogen wird, verhindert sicher das Verstärkungsrohr 11.

Der neue Instrumentensatz vermag die Erfolgsquote beim uterinen Embryonentransfer deutlich zu steigern. Die Verletzungsgefahr für das Endometrium 21 einerseits und den zu übertragenden Embryo andererseits sind bei seiner Verwendung äußerst gering. Außerdem verhindert das Verstärkungsrohrs 11 unangenehme Folgen von Unachtsamkeiten des den Embryonentransfer durchführenden Arztes. Dieser kann sich somit auf das wesentliche, nämlich das verletzungsfreie Einführen des Katheterführungsrohrs und des Katheters selbst vollständig konzentrieren.

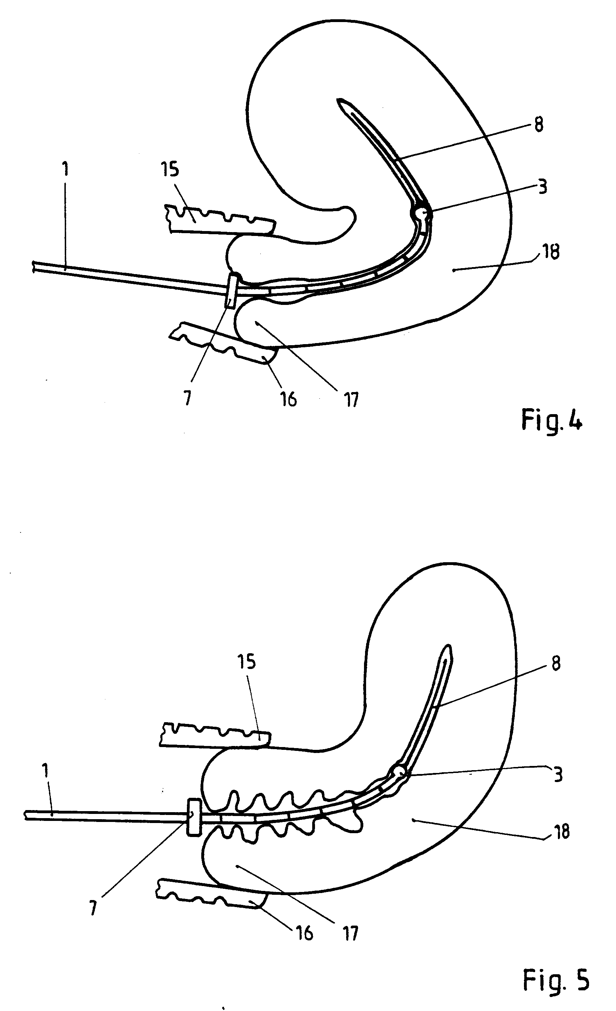

Überraschenderweise läßt sich der neue Instrumentensatz auch erfolgreich bei normalerweise kaum zu überwindenden Problemfällen erfolgreich einsetzen. In Figur 4 ist das eingeführte Katheterführungsrohr 1 mit vorgeschobenem Katheter 8 bei vorliegender Hyperflexio uteri dargestellt. Die Krümmungen 2, 26 des Katheterführungsrohrs 1 und des Katheters 8 sind dafür ursächlich, daß auch stark gekrümmte Oberflächen des Endometriums 21 nicht verletzt werden bzw. ein Einführen des Katheterführungsrohrs 1 und des Katheters 8 unmöglich machen.

In Figur 8 ist der Problemfall eines zerklüfteten Zervikalkanals dargestellt. Hier sind die Kugel 3 und die Krümmung 2 des Katheterführungsrohrs dafür verantwortlich, daß dennoch ein uteriner Embryonentransfer oder eine intrauterine Insemination vorgenommen werden kann.

### Bezugszeichenliste:

- 1: = Katheterführungsrohr
- 2: = Krümmung
- 3: = Kugel
- 4: = distale Öffnung
- 5: = Kupplungsstück
- 6: = proximale Öffnung
- 7: = Marke
- 8: = Katheter
- 9: = Schlauch
- 10: = Spritzenanschluß
- 11: = Verstärkungsrohr
- 12: = Skala
- 13: = distales Ende
- 14: = Spekulum
- 15: = Oberlöffel
- 16: = Unterlöffel
- 17: = Portio
- 18: = Uterus
- 19: = Kugelzange
- 20: = Halterung
- 21: = Endometrium
- 22: = Spritze
- 23: = Flüssigkeit
- 24: = Skala
- 25: = Gegenstück
- 26: = Krümmung

## Patentansprüche

1. Instrumentensatz für den uterinen Embryonentransfer und für die intrauterine Insemination mit einem Katheterführungsrohr (1) und einem in seinem proximalen Bereich mit einem Verstärkungsrohr (11) aus Metall versehenen Katheter (8), dadurch gekennzeichnet, daß das Katheterführungsrohr (1) in seinem distalen Bereich eine leichte Krümmung (2) aufweist, daß das Katheterführungsrohr (1) an seinem distalen Ende mit einer Kugel (3) abschließt und daß das Verstärkungsrohr (11) aus Metall den Katheter (8) umschließt.

2. Instrumentensatz nach Anspruch 1, dadurch gekennzeichnet, daß das Katheterführungsrohr (1) einstückig aus Teflon und insbesondere in seinem distalen Bereich flexibel ausgebildet ist.

3. Instrumentensatz nach einem der Ansprüche 1 oder 2, dadurch gekennzeichnet, daß der Außendurchmesser der Kugel (3) mehr als 2-mal vorzugsweise 3 - 4-mal so groß ist wie der Innendurchmesser des Katheterführungsrohrs (1) an dessen distaler Öffnung (4).

4. Instrumentensatz nach einem der Ansprüche 1 bis 3, dadurch gekennzeichnet, daß der Katheter (8) in seinem distalen Bereich eine der Krümmung (2) des Katheterführungsrohrs (1) entsprechende Krümmung (26) aufweist.

5. Instrumentensatz nach einem der Ansprüche 1 bis 4, dadurch gekennzeichnet, daß der Katheter (8) in seinem proximalen Bereich eine Skala (12) aufweist, die in Verbindung mit der proximalen Öffnung (6) des Katheterführungsrohrs (1) die relative Lage des distalen Endes (13) des Katheters (8) zu der distalen Öffnung des Katheterführungsrohrs (1) anzeigt.

6. Instrumentensatz nach einem der Ansprüche 1 bis 5, dadurch gekennzeichnet, daß das Verstärkungsrohr (11) aus Edelstahl ausgebildet ist und in dem Spritzenanschluß (10) des Katheters (8) gelagert ist.

7. Instrumentensatz nach einem der Ansprüche 1 bis 6, dadurch gekennzeichnet, daß der Katheter (8) einen Schlauch (9) aus poliertem Kunststoff aufweist und an seinem distalen Ende (13) abgerundet ausgebildet ist.

8. Instrumentensatz nach einem der Ansprüche 1 bis 7, dadurch gekennzeichnet, daß das Katheterführungsrohrs (1) in seinem distalen Bereich eine Skala und eine auf der Skala verschiebbare Marke (7) aufweist.

9. Instrumentensatz nach Anspruch 1 oder 8, dadurch gekennzeichnet, daß das Katheterführungsrohr (1) an seinem proximalen Ende eine Kupplungshülse aufweist, und daß der Katheter einen Spritzenanschluß mit einem Gegenstück zum Eingriff in die Kupplungshülse aufweist.

10. Instrumentensatz nach einem der Ansprüche 1 bis 9, dadurch gekennzeichnet, daß das Katheterführungsrohr (1) zur Aufnahme in einer Halterung (20) eines Spekulums (14) mit Kugelzange (19) vorgesehen ist.

## Claims

1. Instrument set for the uterus embryo transfer and intrauterine insemination, having a catheter guide tube (1) and a catheter (8) provided with a metal reinforcing tube (11) in its proximal area, characterized in that the catheter guide tube (1) has a slight bend (2) in its distal area, that the catheter guide tube ends up with a ball (3) at its distal end, and that the reinforcing tube (11) made of metal surrounds the catheter (8).

2. Instrument set according to claim 1, characterized in that the catheter guide tube (1) is constructed in one piece from Teflon and flexibly, in particular, in its distal area.

3. Instrument set according to one of the claims 1 or 2, characterized in that the outside diameter of the ball (3) is more than two times, preferably three or four times, the size of the size of the inside diameter of the catheter guide tube (1) at its distal opening (4).

4. Instrument set according to one of the claims 1 to 3, characterized in that the catheter (8) has a bend (26) in its distal area corresponding to the bend (2) of the catheter guide tube (1).

5. Instrument set according to one of the claims 1 to 4, characterized in that the catheter (8) has a scale (12) in its proximal area which, in conjunction with the proximal opening (6) of the catheter guide tube (1), indicates the position of the distal end (13) of the catheter (8) relative to the distal opening of the catheter guide tube (1).

6. Instrument set according to one of the claims 1 to 5, characterized in that the reinforcing tube (11) is made from special steel and is fitted into the syringe connection (10) of the catheter (8).

7. Instrument set according to one of the claims 1 to 6, characterized in that the catheter (8) has a flexible tube (9) made from polished synthetic material and is round off at its distal end (13).

8. Instrument set according to one of the claims 1 to 7, characterized in that the catheter guide tube (1) has a scale in its distal area and a movable marker (7) is provided on the scale.

9. Instrument set according to claim 1 or claim 8, characterized in that the catheter guide tube (1) has a connecting sleeve at its proximal end, and that the catheter has a syringe connection with a counterpart for being plugged into the connecting sleeve.

10. Instrument set according to one of the claims 1 to 9, characterized in that the catheter guide tube (1) is provided for being received in a holder (20) of a speculum (14) having a ball forceps (10).

## Revendications

1. Jeu d'instruments pour le transfert utérin d'embryons et pour l'insémination intra-utérine, comportant un tube de guidage de cathéter (1) et un cathéter (8), pourvu dans sa zone proximale, d'un tube de renfort (11) en métal, caractérisé en ce que le tube de guidage de cathéter (1) présente, dans sa zone distale, une légère courbure (2), en ce que le tube de guidage de cathéter (1) se termine, à son extrémité distale, par une sphère (3) et en ce que le tube de renfort (1) en métal entoure le cathéter (8).

2. Jeu d'instruments sel on la revendication 1, caractérisé en ce que le tube de guidage de cathéter (1) est réalisé d'une seule pièce en Téflon (marque déposée) et est flexible, en particulier dans sa zone distale.

3. Jeu d'instruments selon l'une des revendications 1 ou 2, caractérisé en ce que le diamètre extérieur de la sphère (3) est plus de deux fois, de préférence trois à quatre fois aussi grand que le diamètre intérieur du tube de guidage de cathéter (1), à son ouverture distale (4).

4. Jeu d'instruments selon l'une des revendications 1 à 3, caractérisé en ce que dans sa zone distale, le cathéter (8) présente une courbure (26) correspondant à la courbure (2) du tube de guidage de cathéter (1).

5. Jeu d'instruments selon l'une des revendications 1 à 4, caractérisé en ce que le cathéter (8) présente, dans sa zone proximale, une échelle (12), qui indique, en liaison avec l'ouverture proximale (6) du tube de guidage de cathéter (1), la position relative de l'extrémité distale (13) du cathéter (8), par rapport à l'ouverture distale du tube de guidage de cathéter (1).

6. Jeu d'instruments selon l'une des revendications 1 à 5, caractérisé en ce que le tube de renfort (11) est en acier spécial et est monté dans le raccord de seringue (10) du cathéter (8).

7. Jeu d'instruments selon l'une des revendications 1 à 6, caractérisé en ce que le cathéter (8) présente un tuyau (9) en matière plastique poli et est arrondi à son extrémité distale (13).

8. Jeu d'instruments selon l'une des revendications 1 à 7, caractérisé en ce que le tube de guidage de cathéter (1) présente, dans sa zone distale, une échelle et une marque (7) déplaçable sur l'échelle.

9. Jeu d'instruments selon les revendications 1 ou 8, caractérisé en ce que le tube de guidage de cathéter (1) présente un manchon d'accouplement à son extrémité proximale, et en ce que le cathéter comporte un raccord de seringue avec une contre-pièce pour l'engagement dans le manchon d'accouplement.

10. Jeu d'instruments selon l'une des revendications 1 à 9, caractérisé en ce que le tube de guidage de cathéter (1) est prévu pour être logé dans une fixation (20) d'un spéculum (14) avec pince à sphère (19).
